# EUROPEAN PATENT APPLICATION

(11) **EP 4 299 010 A1**
(43) Date of publication of application: **03.01.2024**
(21) Application number: 22192629.8
(22) Date of filing: 29.08.2022
(51) Int. Cl.: A61B 8/00, A61B 8/08

(54) **OBTAINING ULTRASOUND IMAGES OF A MOVING ORGAN**

(30) Priority: 27.06.2022 WO PCT/CN2022/101522
(71) Applicant: Koninklijke Philips N.V., 5656 AG Eindhoven (NL)
(72) Inventor: XU, Jingping, Eindhoven (NL); LI, Lin (Happy), Eindhoven (NL); CHEN, Jin Ning, 5656AG Eindhoven (NL); MENG, Yishuang, 5656AG Eindhoven (NL); BAI, Xiang Hui, 5656AG Eindhoven (NL); WEI, Vivian, 5656AG Eindhoven (NL)
(74) Representative: Philips Intellectual Property & Standards

(57) **Abstract**

A method (300) of obtaining ultrasound images of a moving organ of a subject comprises receiving (302) a first sequence of ultrasound images from an ultrasound imaging transducer during a first portion of motion of the organ, wherein the first sequence of ultrasound images is obtained with a first imaging mode having a first field of view. The method then comprises upon a trigger condition, sending (304) an instruction to the ultrasound imaging transducer to cause the ultrasound imaging transducer to switch to a second imaging mode having a second field of view during a second portion of the motion, wherein the second field of view is wider than the first field of view.

## Description

### FIELD OF THE INVENTION

The disclosure herein relates to obtaining ultrasound images of a moving organ.

### BACKGROUND OF THE INVENTION

Pelvic floor dysfunctions (PFDs) are major conditions that frequently occur in adult women, and carry a significant burden on the quality of life. Incidence of pelvic floor dysfunctions tends to increase with age of the population. Pelvic floor dysfunctions can manifest in different ways such as incontinence, constipation, and prolapsed pelvic organs, amongst other things. Since pelvic floor weakness is frequently generalized and clinically underdiagnosed, medical imaging evaluation is of major importance especially prior to surgical correction. Medical imaging, for example: Ultrasound and Magnetic Resonance Imaging, MRI, is widely accepted in clinical practice. Pelvic floor ultrasound or trans-perineal ultrasound (TPUS) is described in the following references: AIUM&IUGA, 'AIUM/IUGA Practice Parameter for the Performance of Urogynecological Ultrasound Examinations', Journal of Ultrasound Medicine, 2019, Vol. 34:851-864; and Abbas Shobeiri S (Editor), "Practical Pelvic Floor Ultrasonography": A multicompartmental Approach to 2D/3D/4D Ultrasonography of the Pelvic Floor, Springer International Publishing AG, 2017 (2nd).

In some pelvic floor examinations, the subject is asked to perform a Valsalva maneuver in which the subject is asked to push out (or bear down) on her pelvic floor muscles as hard as she can for around 10 seconds. It is important that the patient performs her maximal effort. When the doctor tells the subject to start, the aim is for the subject to start pushing as hard as she can and keep pushing as much as possible until the doctor indicates that the subject can relax. A Valsalva maneuver causes downward pressure on the pelvic floor muscles, causing distention of the Levator Ani (LA) muscles. This allows the extent of the Levator Hiatus (LH) to be measured. The maximum distention of the levator ani muscles during a Valsalva maneuver is a key diagnostic indicator of pelvic floor dysfunction, with higher distention linked to pelvic floor weakness. The Valsalva maneuver is discussed further in the paper by Francisco J.O, et.al, 'The time factor in the assessment of prolapse and levator ballooning', Int Urogynecol J, 2012, Vol. 23: 175-178.

### SUMMARY OF THE INVENTION

As noted above, pelvic floor ultrasound examinations can be used to assess pelvic floor function. Useful parameters related to pelvic muscle properties can be obtained from i) 2D static and dynamic imaging (for example: length/width/depth/area of pelvic floor muscles) or ii) static 3D imaging and iii) 4D ultrasound (dynamic 3D) during a Valsalva maneuver.

The levator hiatus area at the maximal pressure during a Valsalva maneuver is an important diagnostic index in pelvic floor ultrasound. In order to measure the levator hiatus area at the maximum point of distention, the optimal volume frame from 4D ultrasound sequence during the Valsalva maneuver (corresponding to the maximum pressure applied by the subject) is currently generally selected manually by the sonographer. There can be more than 20 volume frames from the whole Valsalva maneuver process, and the total number of frames will depend on the duration of the Valsalva maneuver (average time of 9.4 seconds, and range from 5 seconds to 18 seconds) and volume frame rate (for example: of the course of Valsalva maneuver is around 14 second, and frame rate is 2 Hz, then total volume frames is 28 frames).

Dynamic 4D ultrasound acquisitions are helpful for capturing the motion of the Valsalva maneuver. For depth set around 8 cm and fixed sweep angle as well as suitable line density in 2D ultrasound images, there is a trade-off between volume frame rate and field of view angle for a machine-controlled 1D probe. The number of frames that can be captured is limited by the field of view and the depth. Ultrasound involves sending sound waves through the body and receiving reflections from different tissues. Deeper images therefore take longer to acquire as acquisition is limited by the travel time of the sound waves through the body and thus the time it takes to reach an object and reflect back again. 1D probes work by emitting a sequence of beams, each beam sampling a different portion of the angle being sampled. Wider fields of view require higher numbers of beams (each of which has to travel through the tissue and be received in isolation from other beams) thus, a wider field of view also reduces the frame rate. There is thus a trade-off between field of view, depth of image acquisition, and the frame rate that can be acquired.

It is an object of embodiments herein to improve on this situation to enable motion of an organ to be captured with a suitably wide-field image. The invention is defined by the independent claims. The dependent claims define advantageous embodiments.

According to embodiments herein, in a first aspect there is a computer implemented method of obtaining ultrasound images of a moving organ of a subject. The method comprises: receiving a first sequence of ultrasound images from an ultrasound imaging transducer during a first portion of motion of the organ, wherein the first sequence of ultrasound images are obtained with a first imaging mode having a first field of view; and, upon a trigger condition, sending an instruction to the ultrasound imaging transducer to cause the ultrasound imaging transducer to switch to a second imaging mode having a second field of view during a second portion of the motion, wherein the second field of view is wider than the first field of view.

According to a second aspect, there is an apparatus for use in obtaining ultrasound images of a moving organ of a subject. The apparatus comprises: a memory comprising instruction data representing a set of instructions; and a processor configured to communicate with the memory and to execute the set of instructions. The set of instructions, when executed by the processor, cause the processor to perform the method of the first aspect.

According to a third aspect, there is a computer program product comprising a computer readable medium, the computer readable medium having computer readable code embodied therein, the computer readable code being configured such that, on execution by a suitable computer or processor, the computer or processor is caused to perform the method of the first aspect.

Thus, a first field of view may be used to capture a first portion of the motion of the object and a second, wider, field of view may be initiated following a trigger. This enables the motion in the first portion to be captured at a high frame rate in order to clearly capture the motion (e.g. in 4D), and enable a switch to occur to obtain a wider-angle image when a particular point in the motion is reached. This therefore provides a solution when a wide-angle image is required in order capture an image at particular portion/point of the motion but where wide-angle imaging cannot be used throughout as this would reduce the frame rate below a rate at which the motion can be captured in order to accurately determine the trigger point.

These and other aspects will be apparent from and elucidated with reference to the embodiment(s) described hereinafter.

### BRIEF DESCRIPTION OF THE DRAWINGS

Example embodiments will now be described, by way of example only, with reference to the following drawings, in which:
Fig. 1 shows an apparatus according to some embodiments herein;
Fig. 2 shows an ultrasound imaging system according to some embodiments herein;
Fig. 3 shows a method according to some embodiments herein;
Fig. 4 shows two example imaging modes that may be used for the first imaging mode according to some embodiments herein; and
Fig. 5 shows two example imaging modes that may be used for the second imaging mode according to some embodiments herein.

### DETAILED DESCRIPTION OF EMBODIMENTS

As described above, pelvic floor dysfunctions are major conditions that frequently occur in adult women, carry a significant burden on the quality of life, and its incidence tends to increase with senior age of the population.

Pelvic floor ultrasound or trans-perineal ultrasound (TPUS) is well accepted in clinical practice especially for dynamic 4D ultrasound where 3D volumes are obtained for Valsalva maneuver course duration of 6 to 16 seconds (averaged value around 9.5 seconds). For a depth set around 8 cm and a fixed sweep angle as well as suitable line density in 2D ultrasound image acquisition, there is a trade-off between volume frame rate and field of view angle for machine-controlled 1D probes. The field of view angle can be reduced to be smaller angle (for example:69.7 degrees) for the benefit of higher volume frame rate.

However, lower field of view angles may not cover the full area of the levator hiatus. For example, a field of view of 69.7 degrees can only cover the area of the levator hiatus below 30 cm² which is suboptimal for severe pelvic floor conditions where the maximal levator hiatus area during the Valsalva maneuver could be over 64 cm². A field of view angle of 101.7 degrees will be good enough the capture the maximal levator hiatus area during the Valsalva maneuver but will reduce volume frame rate.

In order to keep the same volume frame rate while still obtaining a static-volume 3D wide scan frame that covers the levator hiatus area correctly, it is proposed herein to keep regular 4D acquisition at the beginning (e.g. during a first portion) of the Valsalva maneuver (as the levator hiatus area is relatively small at the start of the Valsalva maneuver) until the maximal effort condition is reached (second portion), then switch the 4D acquisition to static 3D wide scan. In embodiments herein, the switch may be triggered by: a) visual dynamic observation of 3D sequence on the screen by a clinician/ultrasound specialist or b) triggering the 4D/3D mode switch based on a dynamic waveform of the pelvic floor muscle contractions as observed/inferred using, e.g. motion from a sensor array/camera-based positioning sensor array/pressure sensor array. The benefit of this is that by changing the current pelvic floor ultrasound workflow in this manner, accurate levator hiatus area measurements can be acquired from wide-scan 3D volume frames.

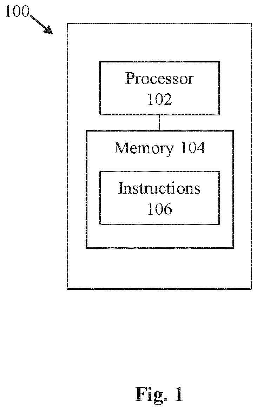

Turning now to Fig. 1, in some embodiments there is an apparatus 100 for use in obtaining ultrasound images of a moving organ. Generally, the apparatus may form part of a computer apparatus or system e.g. such as a laptop, desktop computer or other computing device. In some embodiments, the apparatus 100 may form part of a distributed computing arrangement or the cloud.

The apparatus comprises a memory 104 comprising instruction data representing a set of instructions, and a processor 102 (e.g. processing circuitry or logic) configured to communicate with the memory and to execute the set of instructions. Generally, the set of instructions, when executed by the processor, may cause the processor to perform any of the embodiments of the method 300 as described below.

Embodiments of the apparatus 100 may be for use in obtaining ultrasound images of a moving organ. More specifically, the set of instructions, when executed by the processor, cause the processor to: receive a first sequence of ultrasound images from an ultrasound imaging transducer during a first portion of motion of the organ, wherein the first sequence of ultrasound images are obtained with a first imaging mode having a first field of view; and upon a trigger condition, send an instruction to the ultrasound imaging transducer to cause the ultrasound imaging transducer to switch to a second imaging mode having a second field of view during a second portion of the motion, wherein the second field of view is wider than the first field of view.

The processor 102 can comprise one or more processors, processing units, multi-core processors or modules that are configured or programmed to control the apparatus 100 in the manner described herein. In particular, implementations, the processor 102 can comprise a plurality of software and/or hardware modules that are each configured to perform, or are for performing, individual or multiple steps of the method described herein. The processor 102 can comprise one or more processors, processing units, multi-core processors and/or modules that are configured or programmed to control the apparatus 100 in the manner described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 104 is configured to store program code that can be executed by the processor 102 to perform the method described herein. Alternatively or in addition, one or more memories 104 may be external to (i.e. separate to or remote from) the apparatus 100. For example, one or more memories 104 may be part of another device. Memory 104 can be used to store the first sequence of images and/or any other information or data received, calculated or determined by the processor 102 of the apparatus 100 or from any interfaces, memories or devices that are external to the apparatus 100. The processor 102 may be configured to control the memory 104 to store the first sequence of images.

In some embodiments, the memory 104 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 1 only shows the components required to illustrate this aspect of the disclosure and, in a practical implementation, the apparatus 100 may comprise additional components to those shown. For example, the apparatus 100 may further comprise a display. A display may comprise, for example, a computer screen, and/or a screen on a mobile phone or tablet. The apparatus may further comprise a user input device, such as a keyboard, mouse or other input device that enables a user to interact with the apparatus, for example, to provide initial input parameters to be used in the method described herein. The apparatus 100 may comprise a battery or other power supply for powering the apparatus 100 or means for connecting the apparatus 100 to a mains power supply.

In some embodiments, the apparatus is incorporated into an ultrasound imaging system. For example, an ultrasound imaging system may comprise the apparatus 100 and a display to display the first sequence of images and/or a second image or images obtained with the second ultrasound imaging mode.

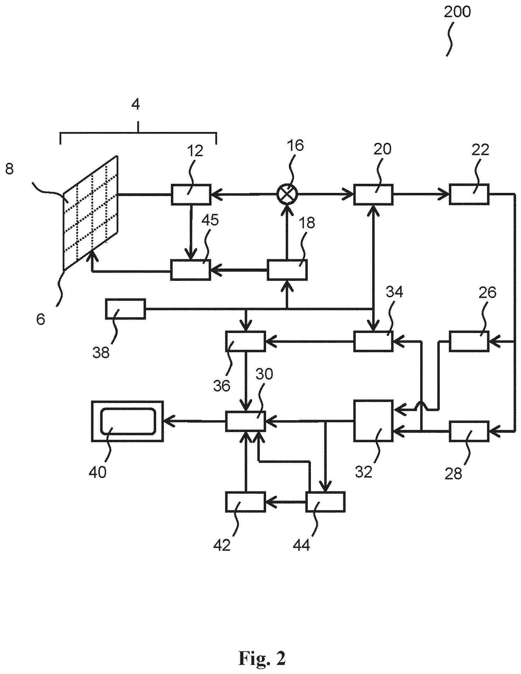

An ultrasound imaging system may further comprise other components, such as those associated with obtaining and processing ultrasound image data. An example ultrasound imaging system 200 is shown in Fig. 2. Ultrasound system 200 comprises an array transducer probe 4 which has a transducer array 6 for transmitting ultrasound waves and receiving echo information. The transducer array 6 may comprise CMUT (capacitive micromachined ultrasonic transducers) transducers; piezoelectric transducers, formed of materials such as PZT (lead zirconate titanate) or PVDF (polyvinylidene fluoride); or any other suitable transducer technology. In this example, the transducer array 6 is a two-dimensional array of transducers 8 capable of scanning either a 2D plane or a three-dimensional volume of a region of interest. In another example, the transducer array may be a 1D array.

The transducer array 6 may be coupled to a micro-beamformer 12 which controls reception of signals by the transducer elements. Micro-beamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

In an alternative embodiment, instead of a micro-beamformer 12, the transducer array may be operated directly by a main system beamformer (not shown in Fig. 2).

The system 200 may further comprise a transmit/receive (T/R) switch 16, which the micro-beamformer 12 can be coupled to and which switches the array between transmission and reception modes. The transmission of ultrasound beams from the transducer array 6 is directed by a transducer controller 18 coupled to the micro-beamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a micro-beamformer) during the transmission mode.

It is noted that in an alternative embodiment, instead of a micro-beamformer 12, the transducer array is operated directly by a main system beamformer, a T/R switch 16 may protect the main beamformer 20 from high energy transmit signals.

In a typical line-by-line imaging sequence, the beamforming system within the probe may operate as follows. During transmission, the beamformer (which may be the micro-beamformer or the main system beamformer depending upon the implementation) activates the transducer array, or a sub-aperture of the transducer array. The sub-aperture may be a one-dimensional line of transducers or a two-dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated, and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above-described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the micro-beamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 2 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B-mode (i.e. brightness mode, or 2D imaging mode) processor 26 and a Doppler processor 28. The B-mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B-mode image. The exact location of the pixel within the image is determined by the location of the associated amplitude measurement along the RF signal and the line (beam) number of the RF signal. B-mode images of such structures may be formed in the harmonic or fundamental image mode, or a combination of both as described in US 6,283,919 (Roundhill et al.) and US 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B-mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B-mode imaging, the brightness of pixel at a given coordinate is proportional to the amplitude of the RF signal received from that location. For instance, the scan converter may arrange the echo signal into a two-dimensional (2D) sector-shaped format, or a pyramidal three-dimensional (3D) image. The scan converter can overlay a B mode structural image with colors corresponding to motion at points in the image field, where the Doppler-estimated velocities to produce a given color. The combined B-mode structural image and color Doppler image is able to depict tissue motion and blood flow within the structural image field. The multi-planar reformatter will convert echoes that are received from points in a common plane in a volumetric region of the body into an ultrasound image of that plane, as described in US 6,443,896 (Detmer). A volume renderer 42 converts the echo signals of a 3D data set into a projected 3D image as viewed from a given reference point as described in US 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as for example: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor may be used for making measurements in the images. The quantification processor may receive input from a user control panel 38.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes, the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

It will be appreciated that the ultrasound image system illustrated in Fig. 2 is merely an example and that an ultrasound image system may comprise different components to those described above.

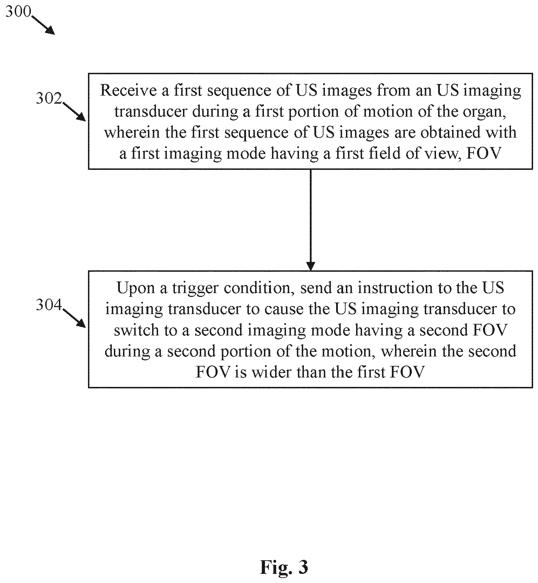

Turning to Fig. 3, there is a computer implemented method 300 for use in obtaining ultrasound images of a moving organ of a subject. Embodiments of the method 300 may be performed, for example by an apparatus such as the apparatus 100 described above, and/or the ultrasound system 200.

Briefly, in a first step 302, the method 300 comprises: receiving a first sequence of ultrasound images from an ultrasound imaging transducer during a first portion of motion of the organ, wherein the first sequence of ultrasound images are obtained with a first imaging mode having a first field of view. In a second step 304 the method comprises, upon a trigger condition, sending an instruction to the ultrasound imaging transducer to cause the ultrasound imaging transducer to switch to a second imaging mode having a second field of view during a second portion of the motion, wherein the second field of view is wider than the first field of view. Typically, the frame rate of the second imaging mode is no higher than that of the first imaging mode, and is lower in most cases. In some embodiments, each of the first and second sequence of ultrasound images is a sequence of 3D ultrasound images. For example, the first imaging mode may be a 4D imaging mode, and the second imaging mode may be a static 3D imaging mode.

As described above, the method relates to obtaining wide-field images of a moving organ. In some embodiments the method relates to obtaining wide-field images of the moving organ at a particular portion or point in the motion.

The method 300 may generally be used in real-time, during an examination of the organ to select an appropriate field of view with which to capture ultrasound images. As such, in step 302 the first sequence of ultrasound images may be obtained from an ultrasound system, in real time as the images are collected.

In some embodiments, the first and/or second imaging modes may be a B-mode imaging mode and the first and second sequence of ultrasound images may be B-mode images. In other embodiments, the first and/or second modes may be doppler modes or elastography modes.

The first sequence of images may be part of an ultrasound image feed or stream. The ultrasound images may otherwise be referred to as ultrasound frames.

The organ may be the muscles associated with the pelvic floor. In some embodiments, the ultrasound images are of pelvic floor muscles of a subject during a Valsalva maneuver. For example, the method 300 may be performed as part of a pelvic floor ultrasound examination.

In other embodiments, the method 300 may be applied to other types of muscle, whereby the size of the muscle changes over contraction and relaxation. It may also be used to obtain a wide field image (e.g. of the chest) at a particular point in the cardiac cycle. The skilled person will appreciate that these are merely examples, and that the method may equally be used to obtain wide-field images at particular points of the motion of other types of organs.

The first sequence of ultrasound images can be a sequence of 3D images, and may be obtained with a 2-Dimensional or a 1-Dimensional transducer.

The first sequence of images may be obtained with a frame rate of at least 4 frames per second (e.g. 4Hz). This allows the motion to be captured in pelvic floor ultrasound since this kind of motion is relatively slow compared to heart beating where a higher frame rate is needed.

In embodiments where the method 300 is performed as part of a pelvic floor examination, the first sequence of ultrasound images may be obtained during a first portion of the Valsalva maneuver and the second sequence of ultrasound images may be obtained during a second portion of the Valsalva maneuver. The first portion may comprise the first half of the Valsalva maneuver, for example, a portion of the Valsalva maneuver during which the levator ani is expanding or ballooning. The second portion of the Valsalva maneuver may comprise a point at which the subject is exerting their maximal (or near maximal) pressure on their pelvic floor.

In such embodiments, the first imaging mode may be a 4D image acquisition mode. The first imaging mode may have a first field of view between about 50 degrees and about 75 degrees. The first imaging mode may have a depth of about 8 cm. In some embodiments, the first field of view is less than about 75 degrees. In some embodiments, the first field of view may be about 69 or about 70 degrees. For example, a field of view of 69.7 degrees and an imaging depth of 8cm leads to a frame rate of 4 frames per second which is sufficient for 4D imaging.

In step 304, responsive to a trigger condition being met, the method comprises sending an instruction (e.g. in the form of a message) to the ultrasound imaging transducer to cause the ultrasound imaging transducer to switch to a second imaging mode having a second field of view during a second portion of the motion, wherein the second field of view is wider than the first field of view.

As an example, the second imaging mode may have a frame rate less than 4 frames per second. In some embodiments, the second field of view is greater than about 75 degrees. In some embodiments, the second field of view is greater than about 100 degrees. For example, the second field of view may be about 101.7 degrees.

In some embodiments, where the first ultrasound image sequence is of the levator ani in order to assess pelvic floor function, the field of view and depth in the second imaging mode are set such that a slice through the 3D image volume covers an area of at least 64cm². This generally corresponds to the maximum viewed distention of the levator ani during a pelvic floor examination and may be set to ensure a wide enough image is obtained that covers the full extent of the levator hiatus.

Generally, the frame rate in the first imaging mode is higher than the frame rate of the second imaging mode. In some embodiments, the frame rate is greater than 4Hz in the first imaging mode and less than 4Hz in the second imaging mode.

In this way, the pelvic floor muscles may be viewed in real-time during the earlier stages of the Valsalva maneuver using a narrower field of view and thus a higher frame rate. This allows the motion to be captured and corresponds to a portion of the Valsalva maneuver where the levator hiatus is smaller. During the second portion of the Valsalva maneuver, during which the maximum distention of the levator ani is reached, the method allows the mode to be switched to a wider field of view so that the full extent of the levator hiatus can be imaged, thus ensuring that appropriate images are obtained for diagnostic purposes. This effectively manages the problem caused by the field of view trade-off with respect to frame rate described above.

It is known that contraction of the pelvic floor muscles during a Valsalva maneuver follows a particular pattern or "waveform". The waveform is generally "S"-shaped. For example, the pressure may initially be low, followed by a period of rapid acceleration, ending in a tailing off of the acceleration as the subject reaches the maximum pressure. Thus, a waveform can be used to determine when the subject has reached their maximum Valsalva maneuver pressure.

Motion, or contraction, of the subject's abdominal muscles; pressure exerted by the subject during the Valsalva maneuver as measured from the subject's abdominal muscles; and the position or change in position of the subject's abdominal muscles (compared to a landmark such as the navel or ribs) are all correlated with the motion of the underlying pelvic floor muscles during the Valsalva maneuver and can thus be used to infer the waveform of the contraction of the pelvic floor muscles. The word waveform is used herein to denote the profile of the pressure, motion or change in position of the muscles over time during the Valsalva maneuver.

Thus, in some embodiments, the method further comprises monitoring a waveform of: motion of the subject's abdominal muscles during the Valsalva maneuver; pressure exerted by the subject during the Valsalva maneuver as measured from the subject's abdominal muscles; and/or position of the subject's abdominal muscles during the Valsalva maneuver compared to a landmark. In such embodiments, the trigger condition may be satisfied or reached, when a pre-defined threshold in (or part of) the waveform is reached.

In some embodiments, the pre-defined threshold indicates (or corresponds to) the distention of the Levator Hiatus being at its maximal distention for the Valsalva maneuver. Distention as used herein refers to the ballooning, dilation or expansion of the muscles associated with the levator ani.

In some embodiments, the predefined threshold may be set based on a reference waveform based on population data, e.g. an average waveform for an average female, for example, as described in academic literature.

In some embodiments, the predefined threshold may be personalized to the subject. For example, a reference e.g. historical waveform may be obtained for the subject. The reference waveform may be the waveform of a Valsalva maneuver performed at the subject's home or during another setting. The pre-defined threshold may then be defined relative to the reference waveform.

In other words, the method 300 may further comprise obtaining a reference waveform of: motion of the subject's abdominal muscles during the Valsalva maneuver compared to a landmark; pressure exerted by the subject during the Valsalva maneuver as measured from the subject's abdominal muscles; and/or position of the subject's abdominal muscles during the Valsalva maneuver; and defining the pre-defined threshold according to the reference waveform. The reference waveform may be previously obtained for the subject during a previous Valsalva maneuver.

The pre-defined threshold can be set at the maximum value of the reference waveform. In some examples, the pre-defined threshold is set at a predefined portion of the maximum value of the reference waveform. For example, the predefined portion may be a percentage (e.g. about 70%, about 80% or about 90%) of the maximum value of the reference waveform.

In other examples, the predefined threshold may be set to a value of the reference corresponding to a turning point in the reference waveform. A turning point corresponds to the curve in the S-shaped waveform and may indicate that the subject is approaching the maximum pressure.

The monitored waveform and/or the reference waveform may be obtained in different ways, for example, using a pressure sensor array positioned on the subject's abdomen, a camera-based positioning sensor array that monitors movements of the subject's abdomen during the Valsalva maneuver and/or a motion sensor array that monitors movements of the subject's abdomen during the Valsalva maneuver.

Turning now to other embodiments, in other embodiments, a waveform is obtained directly from the first sequence of ultrasound images. For example, from measurements of the levator ani and the levator hiatus in the first sequence of ultrasound images. For example, the waveform may represent a geometric property of the levator ani or levator hiatus with respect to time during the Valsalva maneuver. For example, the width, area, circumference, or any other geometric property of the levator hiatus may be measured, in real-time in the first sequence of images and this may be used to trigger the change from the first imaging mode to the second imaging mode. Thus in this way, characteristics of the ultrasound images may be used as the trigger.

For example, geometric properties may be determined in an automated manner using machine learning, ML. For example, a machine learning model may be trained to take an image of the pelvic floor muscles as input and provide an output that can be used to determine the geometric property. For example, a machine learning model may be trained to output the geometric property directly, to output a segmentation of the levator ani, or to provide any other output that can be used to determine the geometric property.

As an example, a You Only Look Once (YOLO) network described in the paper by Redmon et al. (2016) entitled "You Only Look Once: Unified, Real-Time Object Detection*"* may be trained to segment or label (e.g. with geometric information) features in ultrasound images in real-time in ultrasound image feeds.

As another example, a U-net network described in paper "U-Net: Convolutional Networks for Biomedical Image Segmentation" by Olaf Ronneberger et al. can be trained to segment the levator ani from an ultrasound image feed.

The skilled person will appreciate however that these are merely examples, and that other machine learning techniques may equally be used to determine a waveform of the distention of the levator ani muscles from a sequence of ultrasound images of the levator ani muscles.

In other embodiments, step 304 is triggered by a clinician (e.g. a radiologist). For example, the trigger condition may comprise receiving a user input indicating that a maximum point of distention of the Levator Hiatus has been reached by the subject. In this way, the change from 4D imaging to wide-field 3D imaging may be triggered by a clinician.

Thus, in this way, suitable fields of view may be initiated at an appropriate time during the Valsalva maneuver in order to obtain wide-field images suitable for pelvic floor dysfunction analysis.

Turning now to an embodiment, in this embodiment the organ is the pelvic floor muscles, the first portion of motion is a first portion of a Valsalva maneuver and the second portion is a second portion of the Valsalva maneuver. In this embodiment, there is:
- A user interface for clinicians to help the end-user to select an optimal 4D volume sequence and indicator of levator hiatus area change with Valsalva maneuver course;
- An extra-light motion sensor array attached on the abdominal surface of the subject during Valsalva maneuver at home to determining her maximal effort level (and/or to obtain a reference waveform as described above);
- A contactless camera-based optical positioning system to measuring abdominal surface motion of the subject during Valsalva maneuver at home to determine the subject's maximal effort level (and/or to obtain a reference waveform as described above);
- An extra-light pressure sensor array attached on the abdominal surface of the subject during Valsalva maneuver at home to determine their maximal effort level (and/or to obtain a reference waveform as described above);
- The subject is asked to perform a Valsalva maneuver and scanning commences in to the first imaging mode (according to step 302 described above). Any sensor waveform is recorded during the Valsalva maneuver performed during the ultrasound examination (at the hospital) and the personal threshold is determined by her maximal Valsalva maneuver effort level and current waveform for triggering (according to step 304 described above) to static 3D wide scan by software control;
- A module to identify if the pelvic floor ultrasound at Valsalva maneuver is suitable for the subject;
- A module to perform automated key parameter measurement for suitable subject;
- A reporting module to provide information on the representative 2D/3D image and corresponding shapes for the detected levator hiatus with useful levator hiatus dimension.

An example method may be summarised in the following steps:
- Recording typical motion waveform/position waveform/pressure waveform during regular Valsalva maneuver at home for a subject, and any waveform will be used as a reference during regular pelvic floor ultrasound examination at the hospital;

There are two approaches for mode change:
- Display dynamic 3D frame at the screen for end-user to select optimal timing for switching from dynamic 4D mode to wide-scan 3D static mode;
- Display dynamic 3D frame at the screen for end-user and current recorded any waveform is compared to any previous recorded waveform; if the pre-determined threshold (for example: waveform intensity/duration during the typical Valsalva maneuver at the home) is achieved; otherwise, instructing the patient for any Valsalva maneuver until achieve the suitable Valsalva maneuver;
- Reconstructing levator hiatus C-plane from the obtained static 3D volume;
- Measuring the levator hiatus area at the C-plane;
- Measuring the levator hiatus area change from obtained dynamic 4D volume sequence;
- Providing a representative pelvic floor ultrasound image and the levator hiatus area to the end-user.

The trigger condition and the selected mode (first mode or second mode) may be displayed on the screen for the user.

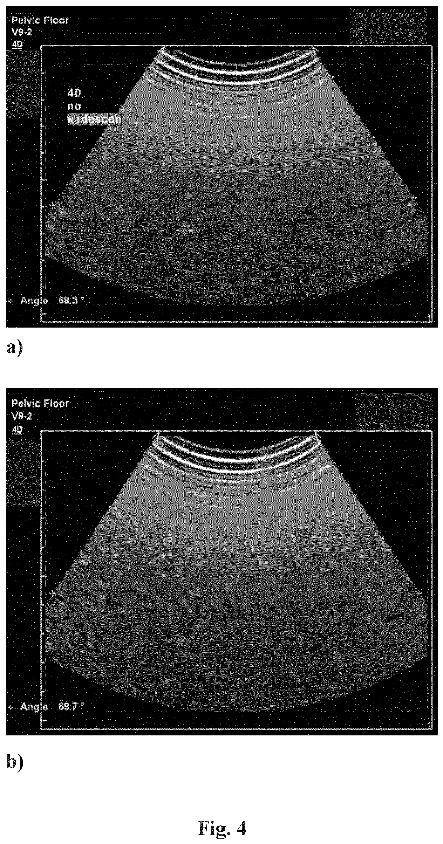

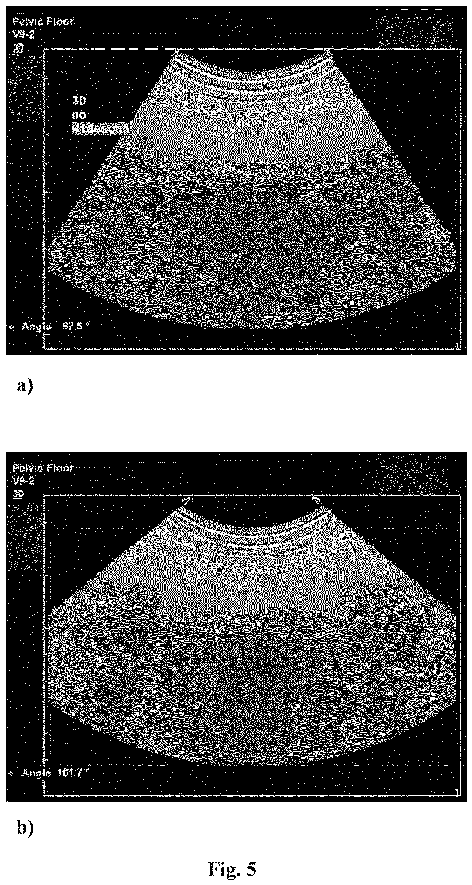

Fig. 4 shows two example options for the first imaging mode (dynamic 4D pelvic floor ultrasound). Image (a) shows non-wide-scan at field of view angle of 68.3 and (b) wide-scan at field of view angle of 69.7. Fig. 5 shows two example options for the second imaging mode (3D pelvic floor ultrasound). Image (a) shows non-wide-scan at field of view angle of 67.5 and (b) wide-scan at field of view angle of 101.7.

In this way, accurate levator hiatus area measurements may be made by acquiring appropriate wide-scan 3D volume frames.

Although the examples above focus on imaging the Valsalva maneuver during a pelvic floor examination, it will be appreciated that the embodiments herein apply equally to imaging of other organs, such as for example, the heart and lungs or the vascular system.

As an example, step 302 may comprise receiving a first sequence of ultrasound images from an ultrasound imaging transducer during a first portion of motion of another muscle type. Then in step 304, upon a trigger condition, such as a maximal or minimal contraction state of the muscle being reached, the method may comprise sending an instruction to the ultrasound imaging transducer to cause the ultrasound imaging transducer to switch to the second imaging mode having a second field of view during a second portion of the motion, wherein the second field of view is wider than the first field of view.

As another example, in an embodiment where the organ is the heart, the trigger condition may be detection of a particular phase of the cardiac cycle (e.g. such as systole or diastole). This may be determined e.g, directly from a first sequence of images of the heart, using a cardiac phase detection process, or manually triggered by a user.

In another embodiment, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. Measures recited in mutually different dependent claims may be advantageously combined. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer implemented method (300) of obtaining ultrasound images of a moving organ of a subject, the method comprising:
receiving (302) a first sequence of ultrasound images from an ultrasound imaging transducer during a first portion of motion of the organ, wherein the first sequence of ultrasound images are obtained with a first imaging mode having a first field of view; and
upon a trigger condition, sending (304) an instruction to the ultrasound imaging transducer to cause the ultrasound imaging transducer to switch to a second imaging mode having a second field of view during a second portion of the motion, wherein the second field of view is wider than the first field of view.

2. A method (300) as in Claim 1, wherein the ultrasound images are of pelvic floor muscles of a subject during a Valsalva maneuver performed by the subject and wherein:
the first sequence of ultrasound images are obtained during a first portion of the Valsalva maneuver and the second sequence of ultrasound images are obtained during a second portion of the Valsalva maneuver.

3. A method (300) as in Claim 2, further comprising monitoring a waveform of:
motion of the subject's abdominal muscles during the Valsalva maneuver;
pressure exerted by the subject during the Valsalva maneuver as measured from the subject's abdominal muscles; and/or
position of the subject's abdominal muscles during the Valsalva maneuver compared to a landmark;
and wherein the trigger condition is satisfied when a pre-defined threshold in the waveform is reached.

4. A method (300) as in Claim 3, wherein the pre-defined threshold indicates that the distention of the levator hiatus, is at its maximal distention for the Valsalva maneuver.

5. A method (300) as in Claim 3 or 4, further comprising obtaining a reference waveform of:
motion of the subject's abdominal muscles during the Valsalva maneuver compared to a landmark;
pressure exerted by the subject during the Valsalva maneuver as measured from the subject's abdominal muscles; and/or
position of the subject's abdominal muscles during the Valsalva maneuver; and
defining the pre-defined threshold according to the reference waveform.

6. A method (300) as in Claim 5, wherein the pre-defined threshold is set:
at a maximum value of the reference waveform; or
at a predefined portion of the maximum value of the reference waveform.

7. A method (300) as in Claim 5 or 6, wherein the pre-defined threshold is set at a value of the reference waveform corresponding to a turning point in the reference waveform.

8. A method (300) as in Claim 5, 6, or 7, wherein the reference waveform was previously obtained for the subject during a previous Valsalva maneuver.

9. A method (300) as in any one of claims 2 to 7, wherein the monitored waveform and/or the reference waveform are obtained using:
a pressure sensor array positioned on the subject's abdomen;
a camera-based positioning sensor array that monitors movements of the subject's abdomen during the Valsalva maneuver; and/or
a motion sensor array that monitors movements of the subject's abdomen during the Valsalva maneuver.

10. A method (300) as in Claim 2 or 3, wherein the waveform is obtained from measurements of the levator hiatus in the first sequence of ultrasound images.

11. A method (300) as in Claim 1, wherein the trigger condition comprises receiving a user input indicating that a maximum point of distention of the Levator Hiatus has been reached by the subject.

12. A method (300) as in any one of the preceding claims, wherein the first field of view is less than about 75 degrees; and/or wherein the second field of view covers an area of at least 64 cm².

13. A method (300) as in any one of the preceding claims, wherein the frame rate in the first imaging mode is higher than the frame rate of the second imaging mode; and/or
wherein the frame rate is greater than 4 Hz in the first imaging mode and less than 4 Hz in the second imaging mode.

14. An apparatus (100) for use in obtaining ultrasound images of a moving organ of a subject, the apparatus comprising:
a memory (104) comprising instruction data representing a set of instructions (106); and
a processor (102) configured to communicate with the memory and to execute the set of instructions, wherein the set of instructions, when executed by the processor, cause the processor to carry out the method of any of the claims 1 to 13.

15. A computer program product comprising computer readable code, the computer readable code being configured such that, on execution by a suitable computer or processor, the computer or processor is caused to perform the method as claimed in any one of claims 1 to 13.
